# EUROPEAN PATENT SPECIFICATION

(11) **EP 3 331 508 B1**
(45) Date of publication and mention of the grant of the patent: **14.08.2024**
(21) Application number: 16824003.4
(22) Date of filing: 01.04.2016
(51) Int. Cl.: A61K 9/00, A61K 9/08, A61K 47/02, A61K 47/12, A61K 47/26, A61K 31/445

(54) **HYPERBARIC SOLUTION INJECTION OF LEVOBUPIVACAINE HYDROCHLORIDE COMPRISING LEVOBUPIVACAINE HYDROCHLORIDE**
INJEKTION VON LEVOBUPIVACAIN-HYDROCHLORID IN HYPERBARISCHE LÖSUNG MIT LEVOBUPIVACAIN-HYDROCHLORID
INJECTION DE SOLUTION HYPERBARE DE CHLORHYDRATE DE LÉVOBUPIVACAÏNE COMPRENANT DU CHLORHYDRATE DE LÉVOBUPIVACAÏNE

(30) Priority: 13.07.2015 IN 2641MU2015
(43) Date of publication of application: 13.06.2018
(73) Proprietor: Neon Laboratories Ltd., Mumbai, Maharashtra 400093 (IN)
(72) Inventor: JOSHI, Neeta, Mumbai 400093 (IN); NARKHEDE, Rahul, Mumbai 400093 (IN)
(74) Representative: Forrest, Stuart
(86) International application number: PCT/IN2016/050101
(87) International publication number: WO 2017/009862

(56) References cited:
- WO-A1-90/00390
- WO-A1-90/00390
- WO-A1-99/25349
- WO-A1-99/25349
- CN-A- 1 628 665
- JP-A- 2002 069 006
- US-A- 6 075 059
- US-A- 6 075 059
- G. A MCLEOD: "Density of spinal anaesthetic solutions of bupivacaine, levobupivacaine, and ropivacaine with and without dextrose", BRITISH JOURNAL OF ANAESTHESIA., vol. 92, no. 4, 6 February 2004 (2004-02-06), GB, pages 547 - 551, XP055348456, ISSN: 0007-0912, DOI: 10.1093/bja/aeh094
- ÖZGÜR YA&GBREVE;AN ET AL: "A comparison of different densities of levobupivacaine solutions for unilateral spinal anaesthesia", BRAZILIAN JOURNAL OF ANESTHESIOLOGY (ENGLISH EDITION), vol. 66, no. 2, 1 March 2016 (2016-03-01), pages 157 - 164, XP055348460, ISSN: 0104-0014, DOI: 10.1016/j.bjane.2014.08.009
- RITA COMPAGNA ET AL: "Comparative study between Levobupivacaine and Bupivacaine for hernia surgery in the elderly", BMC SURGERY, BIOMED CENTRAL, LONDON, GB, vol. 12, no. Suppl 1, 15 November 2012 (2012-11-15), pages S12, XP021121026, ISSN: 1471-2482, DOI: 10.1186/1471-2482-12-S1-S12
- CAPPELLERI GIANLUCA ET AL: "Spinal Anesthesia with Hyperbaric Levobupivacaine and Ropivacaine for Outpatient Knee Arthroscopy: A Prospective, Randomized, Double-Blind Study :", ANESTHESIA AND ANALGESIA, vol. 101, no. 1, 1 July 2005 (2005-07-01), US, pages 77 - 82, XP093064888, ISSN: 0003-2999, DOI: 10.1213/01.ANE.0000155265.79673.56
- CASATI ANDREA ET AL: "A Prospective, Randomized, Double-Blind Comparison of Unilateral Spinal Anesthesia with Hyperbaric Bupivacaine, Ropivacaine, or Levobupivacaine for Inguinal Herniorrhaphy :", ANESTHESIA AND ANALGESIA, 1 November 2004 (2004-11-01), US, pages 1387 - 1392, XP093064891, ISSN: 0003-2999, DOI: 10.1213/01.ANE.0000132972.61498.F1
- MCLEOD G. A. ET AL.: "Density of spinal anaesthetic solutions of bupivacaine, levobupivacaine, and ropivacaine with and without dextrose", BRITISH JOURNAL OF ANAESTHESIA;, vol. 92, no. 4, 6 February 2004 (2004-02-06), pages 547 - 551, XP055348456
- ÖZGÜR Y. ET AL.: "A comparison of different densities of levobupivacaine solutions for unilateral spinal anaesthesia", BRAZILIAN JOURNAL OF ANESTHESIOLOGY, vol. 66, no. 2, pages 157 - 164, XP055348460, [retrieved on 20141126]

## Description

### Technical Field:

The present invention provides hyperbaric injection solution of pharmaceutically acceptable salt of Levobupivacaine. More particularly the invention provides a hyperbaric solution injection of Levobupivacaine Hydrochloride comprising Levobupivacaine Hydrochloride; a baricity adjuster selected from Mannitol and Dextrose to make solution hyperbaric, a base/acid to adjust the pF between 4.0 to 6.0 with water as a vehicle. The invention further relates to process of preparation of a stable hyperbaric injection of Levobupivacaine Hydrochloride.

### Background and prior art:

Levobupivacaine is a local anaesthetic drug belonging to the amino amide group. It is the S-enantiomer of Bupivacaine. Levobupivacaine and its use in solution, for infusion or injection into the epidural or spinal space, or for administration by any of the conventional means for obtaining a nerve or field block, is first disclosed in EP0821588 B1. Currently available (Marketed) formulation of Levobupivacaine Hydrochloride injection is a sterile solution of Levobupivacaine Hydrochloride in water for injections which is isobaric solution. Bupivacaine Hyperbaric solution is also available in market.

Levobupivacaine, the pure S (-)-enantiomer of Bupivacaine, emerged as a safer alternative for regional anesthesia than its racemic form. Levobupivacaine has demonstrated less affinity and strength of depressant effects onto myocardial and central nervous vital centres in pharmacodynamic studies, and has superior pharmacokinetic profile. Levo-enatiomer of bupivacaine appeared to have a safer pharmacological profile than its dextro-partner.

Levobupivacaine had a lower risk of cardiovascular and CNS toxicity than bupivacaine in animal studies.

CelltechGroup Company has launched Levobupivacaine under the trade name 'Chirocaine' in USA for use in surgical and obstetric surgery as local anesthesia and for postoperative pain. Experiments show that Levobupivacaine has better security then Bupivacaine and less central nervous system and cardiac toxicity. There available very few literature on the injectable solutions of levobupivacaine as mentioned below.

CN1628665 discloses Levobupivacaine freeze dried injection and its preparation method. The freeze dried injection comprises pharmaceutically acceptable salt of Levobupivacaine and freeze dryable adjuvant, wherein each preparation unit contains Levobupivacaine pharmaceutically acceptable salt 10-300mg. The freeze dryable adjuvant includes diluent, isotonic conditioning agent, pH modifier.

US6075059A discloses aqueous anesthetic compositions for enhanced anesthesia of peripheral nerves comprising a local anesthetic agent such as Bupivacaine; and a sugar alcohol like mannitol at a concentration of at least 0.1 M.

Freshly prepared Levobupivacaine Hyperbaric solutions with glucose is reported in an article titled "Clinical Characteristics of Spinal Levobupivacaine: Hyperbaric Compared with Isobaric Solution". This article concludes hyperbaric levobupivacaine solutions are more predictable for sensory block level and more effective for surgical procedures with lower abdominal approach; however, optimal dosage needs further investigation.

Levobupivacaine Hyperbaric solution gravitates to dependent areas. Hyperbaric solutions gravitate to the thoracic kyphosis in the supine patient, therefore assuring an adequate level of spinal anesthesia, which is T-6 in the average patient.

Hyperbaric solutions have a greater specific gravity than the cerebrospinal fluid often making the spread of anaesthesia more predictable with greater spread in the direction of gravity.

For surgical procedure performed on patients who are not in the supine position, the baricity of the local anaesthetic solution and gravity are employed to direct the local anaesthetic towards the spinal nerves innervating the surgical site.

Hyperbaric Levobupivacaine solution will provide a more rapid onset and greater spread of anasthesia but a shorter duration of anaesthesia and analgesia. Thus this solution is primarily useful for abdominal surgery procedures of limited duration.

In view of the above, there is still a need to develop hyperbaric solution for injection of Levobupivacaine for making the spread of anaesthesia more predictable with greater spread in the direction of gravity and stable during its shelf life.

Therefore, the object of the invention is to provide a stable Hyperbaric solution for Injection of Levobupivacaine.

### Summary of the invention:

In line with the above objective, the present invention provides stable hyperbaric injection solution of pharmaceutically acceptable salt of Levobupivacaine. The invention provides a hyperbaric solution for injection composition of Levobupivacaine Hydrochloride which comprises; Levobupivacaine Hydrochloride; a baricity adjuster, a base/acid to adjust the pH between 4.0 to 6.0 together with water as a vehicle, wherein the baricity adjuster is selected from dextrose and mannitol, and wherein the composition comprises 10%w/v to 25%w/v of dextrose, or 9%w/v to 10%w/v of mannitol.

In another aspect, the invention provides process of preparation of a stable hyperbaric injection of Levobupivacaine Hydrochloride.

### Detailed description of the invention:

In accordance with the objective, the invention provides stable aqueous hyperbaric solution for injection comprising a pharmaceutically acceptable salt of Levobupivacaine; an acid or base to adjust the pH between 4.0 to 6.0 and a baricity adjuster selected from Mannitol and Dextrose; wherein the composition comprises 10%w/v to 25%w/v of Dextrose or 9 to 10% w/v of mannitol.

Levobupivacaine had shown a lower risk of cardiovascular and CNS toxicity than Bupivacaine in animal studies.

The main rational of the present invention is to provide more rapid onset and greater spread of anasthesia but a shorter duration of anaesthesia and analgesia. Thus this solution is primarily useful for abdominal surgery procedures of limited duration.

The Hyperbaric solutions have a greater specific gravity than the cerebrospinal fluid often making the spread of anaesthesia more predictable with greater spread in the direction of gravity.

According to present invention Levobupivacaine is provided in hyperbaric solution for injection for spinal anaesthesia.

The instant invention provides a hyperbaric solution composition of Levobupivacaine Hydrochloride which comprises Levobupivacaine Hydrochloride; baricity adjuster selected from mannitol and dextrose, a base/ acid to adjust the pH between 4.0 to 6.0 with water as a vehicle, wherein the composition comprises 10%w/v to 25%w/v of dextrose or 9 to 10%w/v of mannitol.

In a preferred embodiment, the hyperbaric solution for injection composition comprises Levobupivacaine Hydrochloride; a base/acid to adjust the pH; Dextrose and water as a vehicle.

According an embodiment, the pH of hyperbaric solution injection is adjusted with base/ acid like potassium hydroxide, sodium hydroxide/ Glacial acetic acid, hydrochloric acid.

In a preferred embodiment, the pH of the hyperbaric solution injection may preferably be adjusted with base/acid like sodium hydroxide/ Hydrochloric acid.

According to the invention, the pH of the hyperbaric solution injection is maintained between 4.0 to 6.0.

In another preferred embodiment, the hyperbaric solution for injection composition comprises Levobupivacaine hydrochloride, a base/acid to adjust the pH; mannitol and water as a vehicle.

In another preferred embodiment, the invention provides a process for preparation of hyperbaric solution injection composition which comprises:
a) Dissolving Dextrose/mannitol in water for injections, followed by addition of Levobupivacaine Hydrochloride;
b) Adjusting the pH of the solution between 4.5 to 6.0; and
c) Making the required volume with cool water for injections.

In an embodiment, the hyperbaric solution may preferably contain 10%w/v to 15%w/v of Dextrose. According to the process the pH of the hyperbaric solution is adjusted with solution of Sodium Hydroxide or Hydrochloric acid.

A hyperbaric solution for injection composition of Levobupivacaine Hydrochloride according to invention comprises 10%w/v to 15%w/v of Dextrose to make the solution hyperbaric.

A hyperbaric solution for injection composition of Levobupivacaine Hydrochloride according to the invention, wherein, the 10%w/v to 15%w/v of Dextrose is dissolved in water for injections.

In another embodiment, the hyperbaric solution for injection composition of Levobupivacaine according to the invention contains 9% w/v to 10%w/v of mannitol.

The hyperbaric solution of Levobupivacaine Hydrochloride according to the invention, wherein, 9%w/v to 10%w/v of mannitol is dissolved in water for injection to make the solution hyperbaric.

Several different trials were conducted & tested for stability by subjecting the hyperbaric solution of Levobupivacaine Hydrochloride prepared in accordance with the invention to accelerated degradation studies (40°C±2°C/75%RH±5% RH). Some of these trials are discussed below in brief.

The following examples, which include preferred embodiments, will serve to illustrate the practice of this invention, it being understood that the particulars shown are by way of examples and for purpose of illustrative discussion of preferred embodiments of the invention.

### Examples

### Example 1: (Reference)

| **Ingredient** | **Quantity/mL** |
|---|---|
| Levobupivacaine Hydrochloride | 5mg |
| Mannitol | 5%w/v |
| Sodium Hydroxide/ Hydrochloric acid | q.s. to pH 4.5 to 6.0 |
| Water For Injections | q.s. to 1ml |

### Procedure:

a) Dissolving Mannitol in a cool water for injections, followed by addition of Levobupivacaine Hydrochloride,
b) adjusting pH of the solution to 4.5 to 6.0 with solution of Sodium Hydroxide or Hydrochloric acid; and
c) Making up the required volume with cool water for injections.

The results are discussed in table 1 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 103.65 | Hyperbaric | Nil | Nil |
| 1M/25°C | 103.81 | Hyperbaric | Nil | Nil |
| 2M/25°C | 106.83 | Hyperbaric | Nil | Nil |
| 3M/25°C | 102.58 | Hyperbaric | Nil | Nil |
| 1M/40°C | 102.29 | Hyperbaric | Nil | Nil |
| 2M/40°C | 101.31 | Hyperbaric | Nil | Nil |
| 3M/40°C | 106.64 | Hyperbaric | Nil | Nil |

### Example 2: (Reference)

**Ingredient Quantity/mL** Levobupivacaine Hydrochloride 5mg Mannitol 9%w/v Sodium Hydroxide/ Hydrochloric q.s. to pH 4.5 to 6.0 acid Water For Injections q.s. to 1ml

### Procedure:

a) Dissolving Mannitol in a cool water for injections, followed by addition of Levobupivacaine Hydrochloride,
b) adjusting pH of the solution to 4.5 to 6.0 with solution of Sodium Hydroxide or Hydrochloric acid and
c) Making up the required volume with cool water for injections.

The results are discussed in table 2 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 102.69 | Hyperbaric | Nil | Nil |
| 1M/25°C | 104.38 | Hyperbaric | Nil | Nil |
| 2M/25°C | 103.75 | Hyperbaric | Nil | Nil |
| 3M/25°C | 101.56 | Hyperbaric | Nil | Nil |
| 1M/40°C | 103.93 | Hyperbaric | Nil | Nil |
| 2M/40°C | 102.73 | Hyperbaric | Nil | Nil |

| | | | | |
|---|---|---|---|---|
| 3M/40°C | 102.87 | Hyperbaric | Nil | Nil |

### Example 3: (Reference)

**Ingredient Quantity/mL** Levobupivacaine Hydrochloride 5mg Dextrose 5.5%w/v Sodium Hydroxide/ Hydrochloric q.s. to pH 4.5 to 6.0 acid Water For Injections q.s. to 1ml

### Procedure:

a) Dissolving Dextrose in a cool water for injections, followed by addition of Levobupivacaine Hydrochloride,
b) adjusting pH of the solution to 4.5 to 6.0 with solution of Sodium Hydroxide or Hydrochloric acid;and
c) Making up the required volume with cool water for injections.

The results are discussed in table 3 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 102.35 | Hyperbaric | Nil | Nil |
| 1M/25°C | 103.89 | Hyperbaric | Nil | Nil |
| 2M/25°C | 102.67 | Hyperbaric | Nil | Nil |
| 3M/25°C | 102.80 | Hyperbaric | Nil | Nil |
| 1M/40°C | 103.92 | Hyperbaric | Nil | Nil |
| 2M/40°C | 103.25 | Hyperbaric | Nil | Nil |
| 3M/40°C | 101.23 | Hyperbaric | Nil | Nil |

### Example 4: (Reference)

| **Ingredient** | **Quantity/mL** |
|---|---|
| Levobupivacaine Hydrochloride | 5mg |
| Dextrose | 8%w/v |
| Sodium Hydroxide/ Hydrochloric acid | q.s. to pH 4.5 to 6.0 |
| Water For Injections | q.s. to 1ml |

### Procedure:

a) Dissolving Dextrose in a cool water for injections, followed by addition of Levobupivacaine Hydrochloride,
b) adjusting pH of the solution to 4.5 to 6.0 with solution of Sodium Hydroxide or Hydrochloric acid; and
c) Making up the required volume with cool water for injections.

The results are discussed in table 4 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 106.22 | Hyperbaric | Nil | Nil |
| 1M/25°C | 106.94 | Hyperbaric | Nil | Nil |
| 2M/25°C | 102.97 | Hyperbaric | Nil | Nil |
| 3M/25°C | 101.90 | Hyperbaric | Nil | Nil |
| 1M/40°C | 106.61 | Hyperbaric | Nil | Nil |
| 2M/40°C | 103.97 | Hyperbaric | Nil | Nil |
| 3M/40°C | 100.49 | Hyperbaric | Nil | Nil |

### Example 5:

| **Ingredient** | **Quantity/mL** |
|---|---|
| Levobupivacaine Hydrochloride | 5mg |
| Dextrose | 10%w/v |
| Sodium Hydroxide/ Hydrochloric acid | q.s. to pH 4.5 to 6.0 |
| Water For Injections | q.s. to 1ml |

### Procedure:

a) Dissolving Dextrose in a cool water for injections, followed by addition of Levobupivacaine Hydrochloride,
b) adjusting pH of the solution to 4.5 to 6.0 with solution of Sodium Hydroxide or Hydrochloric acid and
c) Making up the required volume with cool water for injections.

The results are discussed in table 5 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 103.28 | Hyperbaric | Nil | Nil |
| 1M/25°C | 106.89 | Hyperbaric | Nil | Nil |
| 2M/25°C | 102.45 | Hyperbaric | Nil | Nil |
| 3M/25°C | 102.54 | Hyperbaric | Nil | Nil |
| 1M/40°C | 104.39 | Hyperbaric | Nil | Nil |
| 2M/40°C | 103.74 | Hyperbaric | Nil | Nil |
| 3M/40°C | 102.64 | Hyperbaric | Nil | Nil |

### Example 6:

| **Ingredient** | **Quantity/mL** |
|---|---|
| Levobupivacaine Hydrochloride | 5mg |
| Dextrose | 12.5%w/v |
| Sodium Hydroxide/ Hydrochloric acid | q.s. to pH 4.5 to 6.0 |
| Water For Injections | q.s. to 1ml |

### Procedure:

a) Dissolving Dextrose in a cool water for injections, followed by addition of Levobupivacaine Hydrochloride,
b) adjusting pH of the solution to 4.5 to 6.0 with solution of Sodium Hydroxide or Hydrochloric acid and
c) Making up the required volume with cool water for injections.

The results are discussed in table 6 herein below:

| **Stage** | **Assay %** | **Baricity** | **Chromatography Impurity** | |
|---|---|---|---|---|
| | | | **Individual impurity** | **Total Impurity** |
| | | | **N.M.T. 0.5%** | **N.M.T. 1.0%** |
| Initial | 102.21 | Hyperbaric | Nil | Nil |
| 1M/25°C | 103.54 | Hyperbaric | Nil | Nil |
| 2M/25°C | 104.25 | Hyperbaric | Nil | Nil |
| 3M/25°C | 106.23 | Hyperbaric | Nil | Nil |
| 1M/40°C | 105.21 | Hyperbaric | Nil | Nil |
| 2M/40°C | 102.45 | Hyperbaric | Nil | Nil |
| 3M/40°C | 102.87 | Hyperbaric | Nil | Nil |

## Claims

1. An aqueous hyperbaric solution for injection comprising a pharmaceutically acceptable salt of Levobupivacaine; an acid or base to adjust the pH between 4.0 to 6.0 and a baricity adjuster selected from Mannitol and Dextrose; wherein the composition comprises 10%w/v to 25%w/v of Dextrose or 9 to 10% w/v of mannitol.

2. The hyperbaric solution for injection composition according to claim 1, wherein the acid or base is used to adjust the pH between 4.5 to 6.0.

3. The hyperbaric solution for injection composition according to claim 2, wherein the base is selected from potassium hydroxide, sodium hydroxide and the acid is selected from Glacial acetic acid, hydrochloric acid used to adjust the pH between 4.5 to 6.0.

4. The hyperbaric solution for injection composition according to claim 3, wherein the base is sodium hydroxide or wherein the acid is hydrochloric acid and is used to adjust the pH between 4.5 to 6.0.

5. A process for preparation of hyperbaric solution for injection of Levobupivacaine Hydrochloride which comprises:
a) Dissolving baricity adjuster, Dextrose or mannitol in water for injections, followed by addition of Levobupivacaine Hydrochloride;
b) Adjusting the pH of the solution between 4.5 to 6.0;
c) Making the required volume with cool water for injections; wherein the preparation comprises 10%w/v to 25%w/v of Dextrose or 9 to 10% w/v of mannitol.

6. The process according to claim 5, wherein the pH is adjusted with the solution of Sodium Hydroxide/Hydrochloric Acid.

7. An aqueous hyperbaric solution according to any one of claims 1-4, or aqueous levobupivacaine hydrochloride solution made by a process according to claims 5 or 6, wherein the solution comprises 10%w/v to 25%w/v of Dextrose.

## Patentansprüche

1. Wässrige hyperbare Lösung zur Injektion, die Folgendes umfasst: ein pharmazeutisch verträgliches Salz von Levobupivacain, eine Säure oder Base zur Einstellung des pH auf zwischen 4,0 bis 6,0 und ein Barizität-Einstellungsmittel, das aus Mannitol und Dextrose ausgewählt ist; wobei die Zusammensetzung 10 m/V-% bis 25 m/V-% Dextrose oder 9 bis 10 m/V-% Mannitol umfasst.

2. Zusammensetzung der hyperbaren Lösung zur Injektion nach Anspruch 1, wobei die Säure oder Base zur Einstellung des pH auf zwischen 4,5 bis 6,0 verwendet wird.

3. Zusammensetzung der hyperbaren Lösung zur Injektion nach Anspruch 2, wobei die Base aus Kaliumhydroxid, Natriumhydroxid ausgewählt ist und die Säure aus Eisessig, Salzsäure ausgewählt ist, um zur Einstellung des pH auf zwischen 4,5 bis 6,0 verwendet zu werden.

4. Zusammensetzung der hyperbaren Lösung zur Injektion nach Anspruch 3, wobei die Base Natriumhydroxid ist oder wobei die Säure Salzsäure ist und zur Einstellung des pH auf zwischen 4,5 bis 6,0 verwendet wird.

5. Verfahren zur Zubereitung von hyperbarer Lösung zur Injektion von Levobupivacainhydrochlorid, das Folgendes umfasst:
a) Auflösen von Barizität-Einstellungsmittel, Dextrose oder Mannitol in Wasser für Injektionen, gefolgt von der Zugabe von Levobupivacainhydrochlorid;
b) Einstellen des pH der Lösung auf zwischen 4,5 bis 6,0;
c) Herstellen des erforderlichen Volumens mit kühlem Wasser für Injektionen; wobei die Zubereitung 10 m/V-% bis 25 m/V-% Dextrose oder 9 bis 10 m/V-% Mannitol umfasst.

6. Verfahren nach Anspruch 5, wobei der pH mit der Lösung von Natriumhydroxid/Salzsäure eingestellt wird.

7. Wässrige hyperbare Lösung nach einem der Ansprüche 1-4 oder wässrige Levobupivacainhydrochlorid-Lösung, die durch ein Verfahren nach den Ansprüchen 5 oder 6 hergestellt wird, wobei die Lösung 10 m/V-% bis 25 m/V-% Dextrose umfasst.

## Revendications

1. Solution hyperbare aqueuse pour injection, comprenant un sel pharmaceutiquement acceptable de lévobupivacaïne ; un acide ou une base afin d'ajuster le pH entre 4,0 à 6,0 et un ajusteur de baricité choisi parmi le mannitol et le dextrose ; dans laquelle la composition comprend de 10% p/v à 25% p/v de dextrose ou de 9 à 10% p/v de mannitol.

2. Composition de solution hyperbare pour injection selon la revendication 1, dans laquelle l'acide ou la base est utilisé(e) afin d'ajuster le pH entre 4,5 à 6,0.

3. Composition de solution hyperbare pour injection selon la revendication 2, dans laquelle la base est choisie parmi l'hydroxyde de potassium, l'hydroxyde de sodium et l'acide est choisi parmi l'acide acétique glacial et l'acide chlorhydrique, utilisés afin d'ajuster le pH entre 4,5 à 6,0.

4. Composition de solution hyperbare pour injection selon la revendication 3, dans laquelle la base est l'hydroxyde de sodium ou dans laquelle l'acide est l'acide chlorhydrique, et est utilisé pour ajuster le pH entre 4,5 à 6,0.

5. Procédé de préparation d'une solution hyperbare pour injection de chlorhydrate de lévobupivacaïne, comprenant
a) la solubilisation de l'ajusteur de baricité, du dextrose ou du mannitol dans l'eau pour injections, suivie de l'addition de chlorhydrate de lévobupivacaïne ;
b) l'ajustement du pH de la solution entre 4,5 à 6,0 ;
c) l'ajustement du volume requis avec de l'eau fraîche pour injections, où la préparation comprend de 10% p/v à 25% p/v de dextrose ou de 9 à 10% p/v de mannitol.

6. Procédé selon la revendication 5, dans lequel le pH est ajusté avec la solution d'hydroxyde de sodium/acide chlorhydrique.

7. Solution hyperbare aqueuse selon l'une quelconque des revendications 1-4, ou solution aqueuse de chlorhydrate de lévobupivacaïne préparée par un procédé selon les revendications 5 ou 6, la solution comprenant de 10% p/v à 25% p/v de dextrose.
